# EUROPÄISCHE PATENTANMELDUNG

(11) **EP 0 924 186 A1**
(43) Veröffentlichungstag der Anmeldung: **23.06.1999**
(21) Anmeldenummer: 98121039.6
(22) Anmeldetag: 05.11.1998
(51) Int. Cl.: C07C 67/31, C07C 69/712

(54) **Verfahren zur Herstellung von Aryloxyessigsäureestern**

(30) Priorität: 15.12.1997 DE 19755598
(71) Anmelder: HÜLS AKTIENGESELLSCHAFT, 45764 Marl (DE)
(72) Erfinder: Kaufhold, Manfred, Dr., 45770 Marl (DE); Feld, Marcel, Dr., 51147 Köln (DE)

(57) **Zusammenfassung**

Die Erfindung betrifft ein Verfahren zur Herstellung von Aryloxyessigsäureestern aus Chloressigsäureestern, phenolischen Verbindungen und Alkalialkoholaten, bei dem man die phenolische Verbindung in einem Überschuß an Chloressigsäureester löst, die Lösung erwärmt, ein Alkalialkoholat zusetzt und den entstehenden Alkohol abdestilliert.

## Beschreibung

Die Erfindung betrifft ein Verfahren zur Herstellung von Aryloxyessigsäureestern durch Umsetzung von Chloressigsäureestern mit einer phenolischen Verbindung und einem Alkalialkoholat. Die Umsetzung läßt sich durch das folgende Formelschema beschreiben:

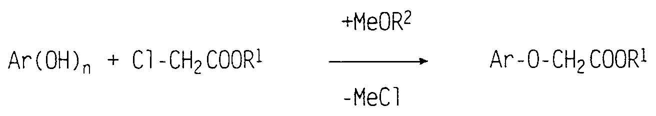

In den Formeln bedeuten
- Ar: einen Arylrest, insbesondere einen Phenyl- oder Naphthylrest, der gegebenenfalls einen oder mehrere inerte Substituenten trägt, beispielsweise Halogenatome, Nitrogruppen, Phenyl oder Benzylgruppen und insbesondere Alkyl- oder Alkoxygruppen -OR³, wobei R³ für einen linearen oder verzweigten Alkylrest mit bis zu 12 Kohlenstoffatomen steht:
- R¹ und R²: unabhängig voneinander einen Alkylrest mit 1 bis 10 Kohlenstoffatomen:
- Me: Natrium oder Kalium; und
- n: für eine ganze Zahl von 1 bis 3.

Aryloxyessigsäureester sind wichtige Zwischenprodukte für die Herstellung von Riechstoffen und Pharmaprodukten. z.B. für einen Penicillin-Typ.

Synthesen von Aryloxyessigsäureestern aus Chloressigsäureestern und phenolischen Verbindungen mit basischen Stoffen als Chlorwasserstoffakzeptoren sind aus der Literatur bekannt. Die übliche und z.B. in US-A 3941889 zitierte Methode ist die von J. Munch-Peterson in Acta Chem. Scand 5.519 [1951] beschriebene. Hierbei wird zunächst eine Lösung von Natriumphenolat in Ethanol hergestellt, und dann wird bei Rückflußtemperatur eine Lösung des Chloressigsäureesters in Ethanol zugegeben. Nach der Umsetzung wird das Natriumchlorid abgetrennt, die große Ethanolmenge abdestilliert und der Rückstand durch Zugabe von Wasser. Etherextraktion und Reindestillation aufgearbeitet. Die Ausbeute beträgt nur 65% bzw., nach DE-OS 23 44 611, 78%. Zudem ist bei diesem Verfahren eine große Menge Ethanol nötig, damit das schwerlösliche Natriumphenolat in Lösung bleibt.

Dieses Problem wird bei dem Verfahren der EP 0796487 dadurch gelöst. daß man die geschmolzene phenolische Verbindung vorlegt und gleichzeitig den Chloressigsäureester und das Alkoholat, in dem entsprechenden Alkohol gelöst, zugibt. Die Abtrennung des Alkalichlorids erfolgt durch Wasserwäsche. Nachteilig bei diesem Verfahren ist, daß die Dosierung sehr genau sein muß, weil bei einem Überschuß an basischer Alkaliverbindung das schwerlösliche Natriumphenolat entsteht. das ausfällt und die Umsetzung erschwert.

Problematisch ist bei beiden genannten Verfahren der Anfall von phenolhaltigem Abwasser, das wegen der Giftigkeit der phenolischen Verbindungen nicht mit vernünftigem Aufwand biologisch gereinigt werden kann.

Eine der Aufgaben der vorliegenden Verbindungen besteht darin, ein Verfahren bereitzustellen, bei dem außer Alkalichlorid kein Feststoff anfällt und bei dem das Alkalichlorid leicht abgetrennt werden kann, ohne daß Abwasser entsteht. Gemäß einer anderen Aufgabe ist ein Verfahren bereitzustellen, das technisch leicht zu realisieren ist. wirtschaftlich arbeitet und Aryloxyessigsäureester von hoher Reinheit in hohen Ausbeuten liefert.

Erfindungsgemäß werden diese Aufgaben gelöst durch ein Verfahren zur Herstellung von Aryloxyessigsäureestern aus Chloressigsäureestern, phenolischen Verbindungen und einem Alkalialkoholat, bei dem man die phenolische Verbindung in einem Überschuß an Chloressigsäureester löst, die Lösung erwärmt, der erwärmten Lösung das Alkalialkoholat zusetzt und den entstehenden Alkohol abdestilliert.

Die erfindungsgemäße Umsetzung entspricht dem obigen Formelschema. Aryloxyessigsäureester und Chloressigsäureester haben deutlich verschiedene Siedepunkte und lassen sich daher durch Rektifikation leicht trennen. Die Aryloxyessigsäureester werden in einer Reinheit von >99% und mit Ausbeuten von >90% gewonnen. Chloressigsäureester sind ausgezeichnete Lösungsmittel für die phenolischen Verbindungen und deren Alkaliphenolate. Der Überschuß an Chloressigsäureester stellt daher sicher, daß zwischenzeitlich kein Alkaliphenolat ausfallt und die Umsetzung stört. Das nach dem Abdestillieren von Aryloxyessigsäureester und verbliebenem Chloressigsäureester zurückbleibende Alkalichlorid ist überraschenderweise nur mit Spuren von Hochsiedern verunreinigt, rieselfähig und gut handhabbar. Es kann ohne aufwendige weitere Reinigung zur Herstellung von neuem Alkalialkoholat oder zu anderen Zwecken in die Chloralkalielektrolyse zurückgeführt werden.

Bevorzugte Chloressigsäureester sind der Ethyl- und insbesondere der Methylester. Von den phenolischen Verbindungen werden ein- oder zwei-kernige Phenole mit ein oder zwei phenolischen Hydroxylgruppen bevorzugt. Die Phenyl-, Biphenyl- oder Naphthylreste können die oben beschriebenen inerten Substituenten enthalten. Beispiele für geeignete phenolische Verbindungen sind neben Phenol selbst α- und β-Naphthol sowie substituierte Phenole und Naphthole. Wie o- und p-Kresol. p-tert.-Butylphenol. Anisol und p-Chlorphenol. Man wendet den Chloressigsäureester und die phenolische Verbindung zweckmäßig im Äquivalentverhältnis von 1.01:1 bis 3:1, insbesondere von 1.05:1 bis 1.5:1 an.

Bevorzugte Alkalialkoholate sind Natrium- und Kaliummethylat sowie die entsprechenden Ethylate. Sie können in äquivalenten Mengen (in bezug auf die phenolische Verbindung) eingesetzt, doch schadet, wie erwähnt, ein geringer Überschuß nicht, weil kein Alkaliphenolat ausfällt. Dieser Überschuß wird zu Alkoxyessigsäureester umgesetzt oder verbleibt unumgesetzt im Reaktionsgemisch. Man kann auch durch gezielte Verwendung eines größeren Überschusses an Alkalialkoholat und Chloressigsäureester in einer Koppelproduktion Arylessigsäureester und Alkoxyessigsäureester herstellen, die ebenfalls wertvolle Zwischenprodukte sind. Im allgemeinen wendet man das Alkalialkoholat und die phenolische Verbindung in einem Äquivalentverhältnis von 3:1 bis 1:1 vorteilhaft von 1.10:1 bis 1.02:1 an. Vorteilhaft ist der Überschuß an Chloressigsäureester größer als der an Alkalialkoholat, jeweils bezogen auf die phenolische Verbindung, so daß nach vollständiger Umsetzung im Reaktionsgemisch kein Alkalialkoholat, sondern restlicher Chloressigsäureester vorhanden ist.

Man führt das erfindungsgemäße Verfahren im allgemeinen ohne Zusatz eines inerten Lösungsmittels durch, doch kann man solche Lösungsmittel mitverwenden, und zwar in Mengen, die im allgemeinen 10 bis 80 Gew.-% betragen, bezogen auf das gesamte Reaktionsgeisch. Geeignete Lösungsmittel sind z.B. aliphatische oder aromatische Kohlenwasserstoffe (wie "Marlotherm S"). Ester und Acetale.

Die Reaktionstemperatur beträgt vorteilhaft 70 bis 150°C, insbesondere 80 bis 120°C. Unterhalb von 70°C ist die Reaktionsgeschwindigkeit nur gering.

Man führt das Verfahren beispielsweise durch, indem man die phenolische Verbindung in dem Chloressigsäureester löst, die Lösung auf 80 bis 85°C erwärmt und das Alkalialkoholat als Lösung in dem betreffenden Alkohol zugibt. Beispielsweise kann man Natriummethylat als 30-gewichtsprozentige Lösung in Methanol zugeben. Während der Zugabe der Lösung destilliert der Alkohol ab. Wenn man ein Alkaliethylat verwendet, arbeitet man zweckmäßig bei einer etwas höheren Temperatur, so daß Ethanol abdestillieren kann. Nach Beendigung der Zugabe des Alkalialkoholats setzt man die Destillation fort, bis die Umsetzung beendet ist. Dann kann man das Reaktionsgemisch auf verschiedene Weise aufarbeiten. Entweder trennt man zunächst das entstandene Alkalichlorid ab. z.B. durch Filtrieren. Zentrifugieren oder Wasserwäsche, und destilliert die verbliebene organische Phase, also Aryloxyessigsäureester und überschüssigen Chloressigsäureester. Vorzugsweise aber destilliert (oder toppt) man, zweckmäßig bei vermindertem Druck, die organische Phase vom entstandenen Alkalichlorid ab. Dies kann z.B. in einem Dünnschichttrockner geschehen, in dem rieselfähiges Alkalichlorid zurückbleibt. Wenn man mit den beschriebenen, geringen Überschüssen an Chloressigsäureester arbeitet, erhält man bei beiden Aufarbeitungsvarianten auch ohne Rektifizierung unmittelbar reinen Aryloxyessigsäureester.

Die Ausbeute an destilliertem Aryloxyessigsäureester mit einer Reinheit von >99% beträgt deutlich über 90%.

Die Erfindung wird durch die folgenden Beispiele weiter erläutert. nicht jedoch in ihrem Anwendungsbereich begrenzt.

### Beispiele

### Beispiel 1

In einer Glasapparatur, die aus einem 2 l-Vierhalskolben mit Rührer. Thermometer. Tropftrichter und einer mit Berlsättel-Füllkörpern gefüllten. 50 cm langen Destillationskolonne mit aufgesetzter Destillationsbrücke besteht, werden 358,1 g (3,3 mol) Chloressigsäuremethylester vorgelegt. Man löst darin 282,3g (3 mol) Phenol und erwärmt die Lösung auf 85°C. Innerhalb von 4 Stunden setzt man der Lösung unter Rühren 558,4g (3,1 mol) einer 30-gewichtsprozentigen Lösung von Natriummethylat in Methanol zu. Hierbei destillieren 450g Methanol ab. Man erniedrigt den Druck auf 600mbar und erhöht die Sumpftemperatur auf 163°C, wobei das restliche Methanol, 31g, abdestilliert.

Danach wird der Druck zunächst auf 130mbar und dann auf 15mbar erniedrigt, und die Sumpftemperatur wird entsprechend auf 177°C bzw. 133°C eingestellt. Es fällt ein Zwischenlauf von 31g mit folgender, mittels GC bestimmten Zusammensetzung an:
- Chloressigsäuremethylester: 19 Gew.-%
- Methoxyessigsäuremethylester: 40 Gew.-%
- Phenol: 11 Gew.-%
- Phenoxyessigsäuremethylester: 13 Gew.-%

Darauf wird die Destillationskolonne entfernt, und man toppt über die Destillationsbrücke Phenoxyessigsäuremethylester vom Natriumchlorid ab. Es fallen 470g Phenoxyessigsäuremethylester mit einer gaschromatographisch bestimmten Reinheit von 99,1% an. Die Ausbeute beträgt 93%. Als Rückstand verbleiben 194g hellgraues Natriumchlorid, das nach einer Analytik mit mehreren Extraktionen nur 0,3 Gew.-% Phenoxyessigsäuremethylester und 1,4 Gew.-% Hochsieder enthält.

### Beispiel 2

Man benutzt einen 500 l-Rührreaktor, auf dem sich eine Destillationsapparatur mit einer 300 cm langen Siebbodenkolonne befindet. In 180kg (1,66 kmol) vorgelegtem Chloressigsäuremethylester löst man 141kg (1.50 kmol) Phenol und verfährt weiter, wie in Beispiel 1 beschrieben. Es werden 279kg (1,55 kmol) 30-gewichtsprozentige Lösung von Natriummethanolat in Methanol eingesetzt. Nach dem Abdestillieren des Zwischenlaufs wird das Sumpfprodukt kontinuierlich in einem Dünnschichttrockner destilliert. Man erhält als Destillat 239kg Phenoxyessigsäuremethylester mit einer gaschromatographisch bestimmten Reinheit von 99,2%. Die Ausbeute beträgt ca. 95%. Das anfallende Natriumchlorid enthält nur 0,1 Gew.-% Phenoxyessigsäuremethylester und 0,9 Gew.-% Hochsieder.

### Beispiel 3

In der in Beispiel 1 beschriebenen Apparatur legt man 358,1 g (3,3 mol) Chloressigsäuremethylester vor, erwärmt ihn auf ca. 55°C und löst darin 450,7 g (3,0 mol) 4-tert.Butylphenol. Die Lösung wird auf 80°C erwärmt. Dann werden innerhalb von 4,5 Stunden 558,4 mol g (3,1 mol) einer 30-gewichtsprozentigen Natriummethylatlösung in Methanol zugegeben, wobei Methanol abdestilliert wird.

Danach wird die Destillation im Vakuum, zuerst bei 400 mbar und schließlich bei 15 bis 7 mbar fortgesetzt. Als Hauptlauf fallen bei einer Kopftemperatur von 155 bis 160°C 637 g 4-tert.Butylphenoxyessigsäuremethylester mit einer Reinheit von 99,1 % an. Die Ausbeute beträgt 93,6 %, bezogen auf 4-tert.Butylphenol.

### Beispiel 4

Man benutzt die in Beispiel 1 beschriebene Temperatur und setzt 200g Wärmeträgeröl "Marlotherm S" (Hüls AG. D-45764 Marl). 358,1g (3,3 mol) Chloressigsäuremethylester und 282,3 g Phenol ein. Die Lösung dieser drei Komponenten wird auf ca. 80°C erwärmt, und dann werden innerhalb von 4,5 Stunden 558,4 g (3,1 mol) einer 30-gewichtsprozentigen Natriummethylatlösung in Methanol zugegeben. Man verfährt weiter wie in Beispiel 1 und erhält 465,6 g Phenoxyessigsäuremethylester mit einer Reinheit von 99,3 % in einer Ausbeute von 93,4 %, bezogen auf Phenol. Der Destillationssumpf, 394,2 g, enthält das entstandene Natriumchlorid in sehr feiner Form und läßt sich gut rühren und pumpen.

Zur Rückgewinnung des Marlotherms wird das Sumpfprodukt in einen 21-Kolben überführt und mit 800 ml Wasser versetzt. Das Natriumchlorid wird aus dem Wärmeträgermittel herausgelöst. Die obere, organische Phase wird abgetrennt, im Vakuum getrocknet und für die nächste Umsetzung in den Reaktor gegeben. Die wäßrige Phase hat einen Kohlenstoffgehalt von 290 mg/l und kann deshalb in das Abwasser entsorgt werden.

## Patentansprüche

1. Verfahren zur Herstellung von Aryloxyessigsäureestern aus Chloressigsäureestern, phenolischen Verbindungen und Alkalialkoholaten. dadurch gekennzeichnet, daß man die phenolische Verbindung in einem Überschuß an Chloressigsäureester löst, die Lösung erwärmt, ein Alkalialkoholat zusetzt und den entstehenden Alkohol abdestilliert.

2. Verfahren nach Anspruch 1, dadurch gekennzeichnet, daß man das Reaktionsgemisch aufarbeitet, indem man zunächst das entstandene Alkalichlorid abtrennt und die verbliebene organische Phase destilliert oder die organische Phase vom Alkalichlorid abdestilliert.

3. Verfahren nach Anspruch 1 oder 2, dadurch gekennzeichnet, daß die Reaktionstemperatur 70 bis 150°C beträgt.

4. Verfahren nach Anspruch 1 oder 2, dadurch gekennzeichnet, daß die Reaktionstemperatur 80 bis 120°C beträgt.

5. Verfahren nach einem der Ansprüche 1 bis 4, dadurch gekennzeichnet, daß der Chloressigsäureester und die phenolische Verbindung im Äquivalentverhältnis von 1.01:1 bis 3:1 angewandt werden.

6. Verfahren nach einem der Ansprüche 1 bis 5, dadurch gekennzeichnet, daß der Chloressigsäureester und die phenolische Verbindung in einem Äquivalentverhältnis von 3:1 bis 1:1 angewandt werden.

7. Verfahren nach einem der Ansprüche 1 bis 6, dadurch gekennzeichnet, daß das Alkalialkoholat und die phenolische Verbindung in einem Äquivalentverhältnis von 3:1 bis 1:1 angewandt werden.

8. Verfahren nach einem der Ansprüche 1 bis 6, dadurch gekennzeichnet, daß das Alkalialkoholat und die phenolische Verbindung in einem Äquivalentverhältnis von 1.10:1 bis 1.02:1 angewandt werden.

9. Verfahren nach einem der Ansprüche 1 bis 8, dadurch gekennzeichnet, daß der Überschuß an Chloressigsäureester größer ist als der Überschuß an Alkalialkoholat, jeweils bezogen auf die phenolische Verbindung.

10. Verfahren nach einem der Ansprüche 1 bis 9, dadurch gekennzeichnet, daß man den Aryloxyessigsäureester in einem Dünnschichttrockner vom Alkalichlorid abtoppt.

11. Verfahren nach enem der Ansprüche 1 bis 10, dadurch gekennzeichnet, daß der Überschuß an Chloressigsäureester größer ist als der an Alkalialkoholat, jeweils bezogen auf die phenolische Verbindung.
